# EUROPÄISCHE PATENTANMELDUNG

(11) **EP 1 186 574 A1**
(43) Veröffentlichungstag der Anmeldung: **13.03.2002**
(21) Anmeldenummer: 01121484.8
(22) Anmeldetag: 07.09.2001
(51) Int. Cl.: C02F 5/10, C02F 3/34, C02F 1/50, A61L 12/08

(54) **Verfahren und Vorrichtung zur dauerhaften Elimination von Biofilmen aus flüssigkeitsführenden Systemen**

(30) Priorität: 08.09.2000 DE 10044652
(71) Anmelder: dolphin aquatec deutschland GmbH, 56070 Koblenz (DE)
(72) Erfinder:
(74) Vertreter: von Bülow, Tam, Dr.

(57) **Zusammenfassung**

Das Verfahren zur dauerhaften Biofilmelimination hat mehrere aufeinanderfolgende Schritte: Als erster Schritt wird eine Grundreinigung der Biofilm-kontaminierter Oberflächen in flüssigkeits- bzw. wasserführenden Systemen auf der Basis eines Reinigers mit enzymatischem Wirkstoff durchgeführt. Mit der Grundreinigung wird eine Auflösung und Zerstörung des Biofilms erreicht. Nach Abschluss der Einwirkung des Reinigers mit enzymatischem Wirkstoff wird dieser durch ein Desinfektionsmittel vollständig aus dem System ausgespült. Mit diesem Desinfektionsmittel wird die Abtötung der aus dem Biofilm freigesetzten, jedoch noch an der freien Oberfläche des flüssigkeitsführenden Systems haftenden Bakterien erreicht. Eine abschließende Spülung mit Wasser beendet die Grundreinigung.

## Beschreibung

Die Erfindung betrifft ein Verfahren zur dauerhaften Elimination von Biofilmen aus flüssigkeitsführenden Systemen und eine Vorrichtung zur Durchführung dieses Verfahrens.

Biofilme werden an flüssigkeits- bzw. wasserbenetzten Oberflächen bevorzugt von gramnegativen Bakterien gebildet. Biofilme können sich in verschiedensten Prozessen und Verfahren, wie der Lebensmittelindustrie (z.B. in Brauereien, Molkereien) oder im medizinischen Bereich (Zahnarztpraxen, HNO-Arztpraxen, Dialyseeinheiten), bilden. Beispielsweise in Arztpraxen, insbesondere Zahnarztpraxen wird Wasser nicht nur an Waschbecken sondern auch am Orte der Behandlung verbraucht. An Letzterem insbesondere zum Betrieb von Turbinen oder als Spülflüssigkeit. Der Wasserverbrauch ist sehr gering, so dass diese Gerätschaften in der Regel nur ungenügend gespült sind. Dies hat zur Folge, dass sich bei den dortigen Wassertemperaturen (Raumtemperatur) Keime bilden. Diese Keime können sich in schädlicher Weise vermehren und insbesondere an den Wänden des Wasserleitungssystems einen sogenannten Biofilm bilden. Dieser Biofilm kann wiederum Keime beherbergen, die sich dort vermehren. Mit den, dem derzeitigen Stand von Wissenschaft und Technik entsprechenden Verfahren sind Biofilme nur unzureichend sicher zu eliminieren.

Mit der Erfindung soll die Sanierung und dauerhafte Elimination von Biofilmen aus flüssigkeitsführenden Systemen, wie z.B. von Modulen und Elementen der Wassergewinnung, Wasserleitung und Wasserspeicherung sowie von Wasserarmaturen, in welchen sich Biofilme bilden können, erreicht werden. Solche Behältnisse sind beispielsweise Tanks, Behältnisse, Rohrleitungen, Schläuche inklusive der dazugehörigen Ventile.

Der Erfindung liegt daher die Aufgabe zugrunde, derart kontaminierte Wasserleitungssysteme, insbesondere solche, die auch Behandlungseinheiten mit umfassen, dauerhaft nachwirkend von den Kontaminationen zu befreien.

Gelöst wird die Aufgabe durch die in den Ansprüchen angegebenen Merkmale.

Gemäß Patentanspruch 1 erfolgt das Verfahren zur dauerhaften Biofilmelimination in mehreren aufeinanderfolgenden Schritten.

Als erster Schritt wird eine Grundreinigung derart Biofilmkontaminierter Oberflächen in flüssigkeits- bzw. wasserführenden Systemen auf Basis eines Reinigers mit enzymatischem Wirkstoff durchgeführt. Mit der Grundreinigung wird eine Auflösung und Zerstörung des Biofilms erreicht.

Nach Abschluss der Einwirkung des Reinigers mit enzymatischem Wirkstoff wird dieser durch ein Desinfektionsmittel vollständig aus dem System ausgespült. Mit diesem Desinfektionsmittel wird die Abtötung der aus dem Biofilm freigesetzten, jedoch noch an der freien Oberfläche des flüssigkeitsführenden Systems haftenden Bakterien erreicht.

Eine abschließende Spülung mit Wasser beendet die Grundreinigung.

Entsprechend der Besonderheiten des jeweiligen Prozesses wird anschließend z.B. bei Trinkwassersystemen im Rahmen einer kontinuierlichen Dauerbehandlung ein Desinfektionsmittel in einer geringen Konzentration zudosiert werden, so dass ein erneuter Biofilm-Aufwuchs dauerhaft unterbunden wird.

Bei Prozessen, welche eine kontinuierliche Zudosierung von Desinfektionswirkstoffen nicht erlauben (z.B. Bereiche der Lebensmittelindustrie), erfolgt eine diskontinuierliche Wiederholung der Grundreinigung in festzulegenden Zeiträumen.

### Verfahrensschritte Grundreinigung:

Im Verfahrensschritt der Grundreinigung wird ein Reiniger mit enzymatischem Wirkstoff mittels einer Dosiervorrichtung dem temperierten Wasser zudosiert, der den vorhandenen Biofilm in der folgenden Einwirkzeit auflöst bzw. zerstört. Die Einwirkzeit liegt entsprechend dem verwendeten Präparat des Reinigers mit enzymatischem Wirkstoff und dessen präparatespezifischer Temperatur in einem Zeitraum von unter 2 Stunden bei einer Konzentration von unter 10 %.

Nach einer Variante der Erfindung ist vorgesehen, dass dem Enzymreiniger ein Neutralreiniger zugegeben wird. Dies kann in der gleichen Konzentration erfolgen. Die Konzentration des Neutralreinigers liegt bevorzugt unter 10 % und vorzugsweise bei 1 %.

Nach einer weiteren Variante der Erfindung ist vorgesehen, dass die Temperatur des Wassers dem der Enzymreiniger bzw. der Neutralreiniger beigegeben wird oder worden ist, auf 30°C bis 60°C gebracht oder gehalten wird.

Anschließend wird der Reiniger mit enzymatischem Wirkstoff durch ein Desinfektionsmittel, welches dem strömenden Wasser mittels der Dosiervorrichtung zugesetzt ist, ausgespült.

Als Desinfektionspräparate können die entsprechend der Trinkwasserverordnung zugelassenen Wirkstoffe, wie z.B. Chlor, Chloramin, Chlordioxyd oder Ozon etc., mit der dem jeweiligen Präparat entsprechenden Einwirkzeit im zu behandelnden System belassen werden. Es können auch sonstige Desinfektionsmittel außerhalb der Trinkwasserverordnung zugegeben werden, wie z.B. Wasserstoffperoxyd, wenn das Wasser nicht der TVO entsprechen muß.

Zur Beendigung der Grundreinigung wird das System mit Wasser bis zum Nachweis der vollständigen Entfernung der Reste des Desinfektionspräparates gespült.

### Verfahrensschritte kontinuierliche Dauerbehandlung:

Für eine kontinuierliche Dauerbehandlung muss aufgrund der prozesstechnischen Rahmenbedingungen (z.B. Trinkwassersysteme) nach Abschluss der Grundreinigung eine dauerhafte Zudosierung eines Desinfektionspräparates (Dauerbehandlungswirkstoff) zulässig sein.

Dabei wird die vorhandene Steuereinheit derart beeinflusst, dass z.B. das Wasser mit einem Dauerbehandlungswirkstoff in geringer Konzentration der Zuleitung langfristig zugegeben wird.

Für die kontinuierliche Dauerbehandlung kommen als Desinfektionsmittel zugelassene Präparate entsprechend der Trinkwasserverordnung in den jeweils zulässigen Konzentrationen zur Anwendung.

Zur Sicherstellung der dauerhaften Wirkung gegenüber Biofilm kann ferner festgelegt werden, dass sämtliche Ventile, Armaturen, Leitungssysteme oder sonstige Module in regelmäßigen Abständen, z.B. wöchentlich, geöffnet werden bzw. eine Durchströmung sichergestellt wird und keine Toträume bestehen bleiben.

### Verfahrensschritte diskontinuierliche Behandlung:

Ist eine kontinuierliche Zudosierung von Desinfektionswirkstoffen aus prozesstechnischen Gründen nicht möglich, so erfolgt eine diskontinuierliche Wiederholung der Grundreinigung in festzulegenden Zeiträumen.

Auch im Rahmen der diskontinuierlichen Behandlung kann zur Verminderung des Biofilm-Wachstums festgelegt werden, dass sämtliche Ventile, Armaturen, Leitungssysteme oder sonstige Module in regelmäßigen Abständen, z.B. wöchentlich, geöffnet werden bzw. eine Durchströmung sichergestellt wird.

Im folgenden wird die Erfindung anhand von Ausführungsbeispielen im Zusammenhang mit der Zeichnung ausführlicher erläutert. Es zeigt:
- Fig. 1: eine Prinzipskizze der Vorrichtung für die Grundreinigung; und
- Fig. 2: eine Skizze der Vorrichtung für die Dauerbehandlung.

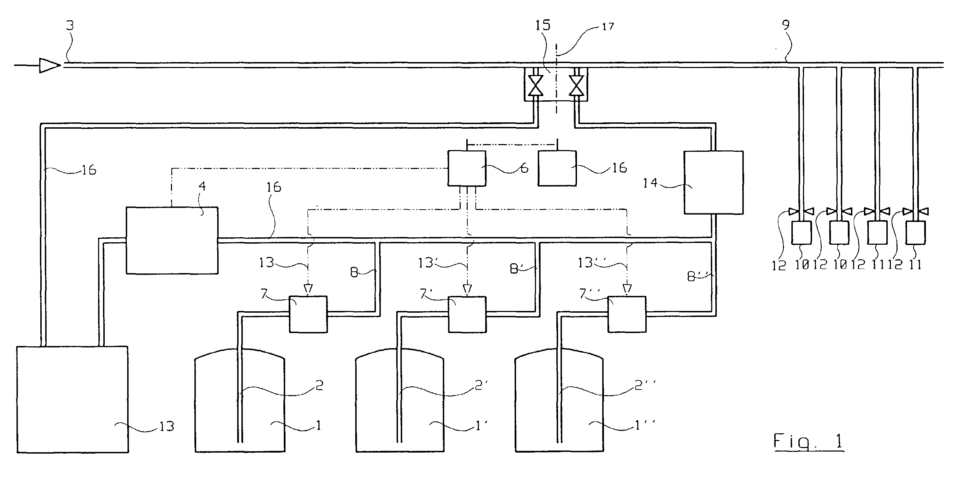

Die Grundreinigung wird normalerweise nur einmal durchgeführt. Die Vorrichtung der Fig. 1 hierzu muß also nicht ständig "vor Ort" bleiben und ist daher auf einem mobilen Gerät montiert, das folgende Einzelaggregate aufweist:

Ein in eine Wasserleitung 3 eingebauter Anschlußblock 15 mit Ventilen leitet Frischwasser von der Zuleitung 3 in die Vorrichtung, und zwar über einen Anschlußschlauch 16. Das unbehandelte Wasser fließt darüber zu einer Warmwassererzeugungsanlage 13, die das Kaltwasser auf weniger als 60°C erwärmt. Von dort durchströmt das erwärmte Wasser eine Wasservolumenmeßeinheit 4, die mengenproportional Impulse an eine Steuereinheit 6 abgibt. Die Steuereinheit 6 steuert über Steuerleitungen 13, 13', 13'' Dosierpumpen 7, 7', 7" an, die aus zugeordneten Vorratsbehältern 1, 1', 1" über ein Saugrohr 2, 2' bzw. 2'' den Reiniger mit enzymatischem Wirkstoff bzw. den Neutralreiniger im ersten Schritt und das Desinfektionsmittel im zweiten Schritt saugen und es durch Impfleitungen 8, 8', 8'' in die Wasserleitung des Sanierungsgerätes pumpen. Das mit den Wirkstoffen versetzte Wasser wird durch eine Verwirbelungseinrichtung 14 geführt, die das Wasser und die Wirkstoffe vermischt, und gelangt durch eine Schlauchleitung zum Anschlußblock 15 zurück. Diese Schlauchleitung ist der Wasserausgang des Sanierungsgerätes und gleichzeitig die Wasserzuführung zur Wasserversorungsleitung 9 des Biofilm-kontaminierten flüssigkeitsführenden Systems. Mittels einer Funkfernbedienung 16 kann der gesamte Betriebszustand des Sanierungsgerätes mit all seinen Einstellungsparametern abgelesen und gesteuert werden. Die Funkfernbedienung 16 ist über eine Funkstrecke mit der Steuereinheit 6 verbunden. Unter Funkstrecke ist jegliche Nachrichtenverbindung zu verstehen, also auch beispielsweise eine Infrarot-Verbindung. Selbstverständlich kann die Funkverbindung wahlweise auch durch eine Kabelverbindung ersetzt werden.

Wie durch die gestrichelte Linie 17 im Anschlußblock 15 angedeutet, fließt das Wasser aus der Zufuhrleitung 3 vollständig durch das beschriebene Gerät und von dort in die Leitung 9, während die direkte Verbindung zwischen den Leitungsabschnitten 3 und 9 unterbrochen ist.

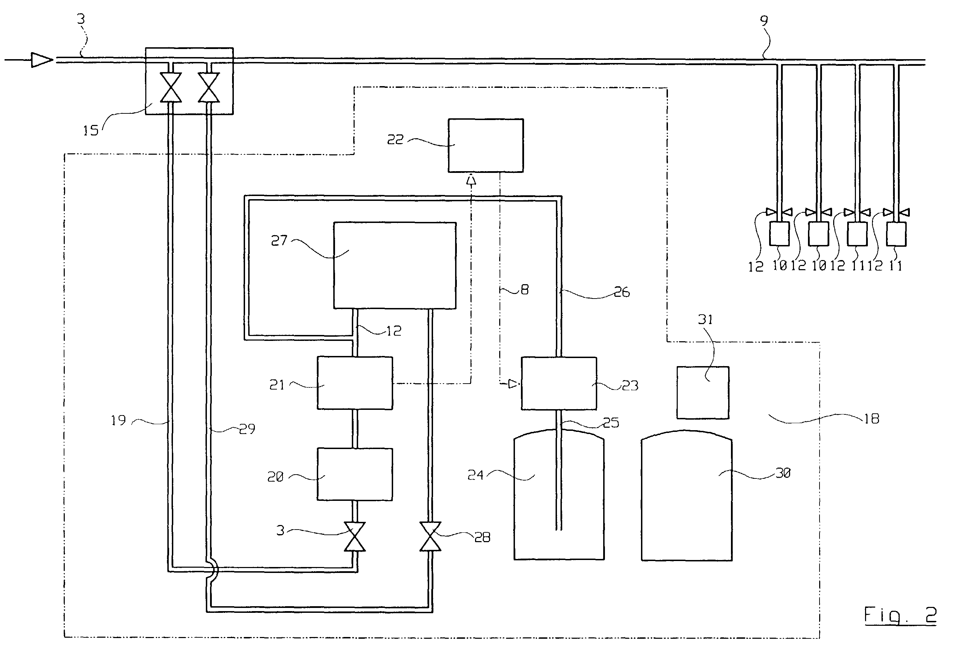

Fig. 2 zeigt die Vorrichtung zur Dauerbehandlung, die ständig "vor Ort" bleibt. Diese Vorrichtung wird an ein zuvor mit der Grundreinigung saniertes Wasserversorgungssystem angeschlossen. Diese Dauerbehandlungseinheit ist in ihrer Gesamtheit mit dem Bezugszeichen "18" bezeichnet. Sie wird über den Anschlußblock 15 an die Frischwasserleitung 3 angeschlossen und fließt von dort über eine Leitung 19 und ein Absperrorgan 3 durch eine Sicherheitsarmatur 20 entsprechend den gültigen technischen Vorschriften bezüglich des auf die abzusichernde Stoffklasse des Dauerbehandlungswirkstoffes. Von dort durchströmt das Wasser eine Wasservolumenmeßeinheit 21, die mengenproportionale Impulse an eine Steuereinheit 22 abgibt. Die Steuereinheit 22 steuert wiederum eine Dosierpumpe 23, die aus einem Vorratsbehälter 24 über ein Saugrohr 25 den Dauerbehandlungswirkstoff saugt und durch eine Impfleitung 26 einer Verwirbelungseinrichtung 27 zuführt, in der das Frischwasser vom Ausgang der Wasservolumenmeßeinheit 21 und der Dauerbehandlungswirkstoff vermischt werden. Von dort durchströmt das mit dem Dauerbehandlungswirkstoff versetzte Wasser ein Absperrorgan 28 und gelangt über einen Leitungsanschluß 29 und den Anschlußblock 15 in das Wasserversorgungsleitungssystem 9. Auch hier kann mit einer Funkfernbedienung 30 der gesamte Betriebszustand der Dauerbehandlungseinheit mit all seinen Einstellungsparametern abgelesen werden. Diese Funkfernbedienung 30 ist ebenfalls funkmäßig mit der Steuereinheit 22 verbunden, wobei auch hier die Funkverbindung wahlweise durch eine Kabelverbindung ersetzt werden kann. In der Dauerbehandlungseinheit 18 kann auch noch ein Reservebehälter 31 mit dem Dauerbehandlungswirkstoff Platz finden.

Die Steuereinheit 22 speichert sämtliche Wasserverbrauchsmengen mit Zeitangaben sowie Betriebszustände, wie z.B. Behälterreservemeldung, Behälterleermeldung, Stromausfall etc. Die Wassermengenerfassung ist von 1,8 Liter bis 3500 Liter pro Stunde möglich.

Der Prozeßablauf der Grundreinigung arbeitet wie folgt:

Zur Grundreinigung wird der Behälter 1 zunächst mit dem Reiniger mit enzymatischem Wirkstoff in flüssiger Form gefüllt. Es handelt sich dabei bevorzugt um eine 1 %-ige Lösung. Bevorzugt liegt die Konzentration des Enzymreinigers im flüssigkeitsführenden System bei unter 10 %.

In einer Variante der Erfindung ist vorgesehen, dass zusätzlich zum Reiniger mit enzymatischem Wirkstoff ein Neutralreiniger (aus Behälter 1') in das flüssigkeitsführende System injiziert wird. Die Konzentration des Neutralreinigers beträgt weniger als 10 % und bevorzugt 1 %.

In einer weiteren Variante der Erfindung erfolgt die Zugabe des Reinigers mit enzymatischem Wirkstoff bzw. Neutralreinigers in temperiertes Wasser. Die Temperatur des Wassers bevorzugt mindestens 30°C und nicht mehr als 60°C. Die Erwärmung des Wassers erfolgt durch die Warmwassererzeugungseinrichtung 13 und kann auch nach Injizierung des Reinigers mit enzymatischem Wirkstoff bzw. Neutralreinigers erfolgen.

Sodann wird in der Steuereinrichtung 6 die gewünschte Konzentration eingestellt. Durch Öffnen aller Ventile 12 wird das gesamte, potentiell mit Biofilm belastete System mit der Lösung gefüllt.

Der Reiniger mit enzymatischem Wirkstoff verbleibt dann für einen Zeitraum von kleiner 2 Stunden in dem System.

Gegebenenfalls kann nach Abschluß der Einwirkzeit des Reinigers mit enzymatischem Wirkstoff das gesamte System entleert werden und ein zweites Behandlungsintervall mittels eines Reinigers mit enzymatischem Wirkstoff wiederholt werden.

Im nächsten Verfahrensschritt wird der Inhalt des Vorratsbehälters 3 verwendet. Das in dem Vorratsbehälter 3 befindliche Desinfektionsmittel der Grundreinigung, beispielsweise eine Natriumhypochloridlösung, wird anschließend in das System 9 durch Öffnen aller Ventile 12 eingefüllt. Dieses Desinfektionsmittel bleibt für eine Einwirkzeit kleiner 12 Stunden im gesamten System 9. Nach Abschluß der Einwirkzeit kann das gesamte System 9 durch Öffnen der Ventile 12 mit z.B. Wasser gespült werden.

Zum Spülen des Systems wird über die Steuerung 6 (Pumpen 7, 7', 7'' sind dabei abgeschaltet) Wasser zugeführt, bis das Desinfektionsmittel der Grundreinigung nicht mehr nachweisbar ist.

Der Prozeßablauf der Dauerbehandlung sieht vor, daß kontinuierlich der Dauerbehandlungswirkstoff aus dem Behälter 24 durch die Dosierpumpe 23 dem Wasser zugemischt und dem Rohrleitungssystem 9 und den angeschlossenen Aggregaten 10, 11 zugeführt wird.

## Patentansprüche

1. Verfahren zur Elimination von Biofilmen wasserbenetzter Oberflächen bzw. flüssigkeitsführender Systeme, wie Wasserversorgungsspeicher oder -leitungssystemen, wie Behältnissen, Tanks, Rohrleitungen, Schläuche oder dergleichen, insbesondere von Zahnarztpraxen, HNO-Arztpraxen, Dialyseeinrichtungen oder Lebensmittelbetrieben, **gekennzeichnet durch**
die Durchführung einer Grundreinigung auf der Basis eines Reinigers mit enzymatischem Wirkstoff sowie anschließendem Einsatz eines Desinfektionsmittels der Grundreinigung.

2. Verfahren nach Anspruch 1, **gekennzeichnet durch** folgende Verfahrensschritte:
- Füllen des flüssigkeitsführenden Systems mit Wasser, dem ein Reiniger mit enzymatischem Wirkstoff zum Zerstören eines auf den Oberflächen der Behältnisse, insbesondere des Leitungssystems vorhandenen Biofilms zugegeben wird;
- Einwirken lassen dieser Lösung für eine vorgegebene Zeit;
- Füllen des flüssigkeitsführenden Systems mit Wasser, dem Desinfektionsmittel zugegeben wird;
- Einwirken lassen der Lösung für eine vorgegebene Zeit;
- ggf. Wiederholen dieser Vorgänge.

3. Verfahren nach Anspruch 1 oder 2, **dadurch gekennzeichnet, dass** im Anschluss an die Verfahrensschritte ein Spülen des flüssigkeitsführenden Systems mit Wasser erfolgt, bis keine Reste des Desinfektionsmittels mehr nachweisbar sind.

4. Verfahren nach Anspruch 1 und 2, **dadurch gekennzeichnet,**
**dass** im Anschluss an die Verfahrensschritte nach Anspruch 2 ein Spülen des flüssigkeitsführenden Systems mit Wasser erfolgt, dem dauerhaft ein Dauerbehandlungswirkstoff in der geringeren Konzentration zugegeben wird.

5. Verfahren nach Anspruch 3, **dadurch gekennzeichnet, dass** Grundreinigungen entsprechend Anspruch 2 diskontinuierlich wiederholt werden.

6. Verfahren nach einem oder mehreren der vorhergehenden Ansprüche, **dadurch gekennzeichnet,**
**dass** der Reiniger auf einem enzymatischen Wirkstoff basiert.

7. Verfahren nach einem oder mehreren der vorhergehenden Ansprüche, **dadurch gekennzeichnet,**
**dass** neben dem enzymatischen Wirkstoff zusätzlich auch ein Neutralreiniger verwendet wird.

8. Verfahren nach einem oder mehreren der vorhergehenden Ansprüche, **dadurch gekennzeichnet,**
**dass** die Konzentration des Neutralreinigers im flüssigkeitsführenden System bis zu 10 % beträgt.

9. Verfahren nach einem oder mehreren der vorhergehenden Ansprüche, **dadurch gekennzeichnet,**
**dass** die wässrige Wirkstofflösung, mit welchem das flüssigkeitsführende System behandelt wird, auf eine Temperatur zwischen 30°C und 60°C gebracht wird.

10. Verfahren nach einem oder mehreren der vorhergehenden Ansprüche, **dadurch gekennzeichnet,**
**dass** das Desinfektionsmittel der Grundreinigung eine Chlorverbindung, insbesondere Natriumhypochlorit, Chlordioxid oder elektrolytisch hergestelltes Chlor oder auch Ozon oder Wasserstoffperoxid oder ein anderes Desinfektionsmittel ist.

11. Verfahren nach einem oder mehreren der vorhergehenden Ansprüche, **dadurch gekennzeichnet,**
**dass** die Einwirkzeit des insbesondere in einer unter 10 %igen Verdünnung angewendeten Reinigers mit enzymatischem Wirkstoff während der Grundreinigung unter 2 Stunden beträgt und ggf. mehrfach aufeinanderfolgend angewendet werden kann.

12. Verfahren nach einem oder mehreren der vorhergehenden Ansprüche, **dadurch gekennzeichnet,**
**dass** die Einwirkzeit des Desinfektionsmittels während der Grundreinigung weniger als 12 Stunden beträgt.

13. Verfahren nach einem oder mehreren der vorhergehenden Ansprüche, **dadurch gekennzeichnet,**
**dass** das Desinfektionsmittel der Grundreinigung eine Chlorkonzentration von kleiner 100 mg/l Wasser aufweist.

14. Verfahren nach einem oder mehreren der vorhergehenden Ansprüche, **dadurch gekennzeichnet,**
**dass** das Desinfektionsmittel der Dauerbehandlung einer Chlorkonzentration von weniger als 0,3 mg/l Wasser aufweist.

15. Vorrichtung zur Durchführung der Grundreinigung nach einem oder mehreren der vorhergehenden Ansprüche, **gekennzeichnet durch**
ein an eine Wasserversorgungsleitung (3) anschließbares mobiles Aggregat, das eine Warmwassererzeugungseinrichtung (13), mindestens einen Vorratsbehälter (1) für einen Enzymreiniger, eine Dosierpumpe (7) und eine Impfleitung (8) aufweist, über die der Enzymreiniger und das erwärmte Wasser dem zu sanierenden Rohrleitungssystem (9) zuführbar ist.

16. Vorrichtung nach Anspruch 15, **gekennzeichnet durch** eine Wasservolumenmeßeinheit (4), die mengenproportional Impulse an eine Steuereinheit (6) abgibt, wobei die Steuereinheit entsprechend diesen Impulsen die Dosierpumpe (7) ansteuert.

17. Vorrichtung nach Anspruch 15 oder 16, **gekennzeichnet durch**
eine Verwirbelungseinrichtung (14), die das erwärmte Wasser und den zudosierten Enzymreiniger vermischt.

18. Vorrichtung nach einem der Ansprüche 15 bis 17, **dadurch gekennzeichnet,**
**dass** mehrere Behälter (1, 1', 1'') und eine entsprechende Anzahl von Dosierpumpen (7, 7', 7'') samt Impfleitungen (8, 8', 8'') vorgesehen sind, die unter Steuerung der Steuereinrichtung (6) weitere Zusatzstoffe dem erwärmten Wasser zudosieren.

19. Vorrichtung zur Durchführung der Dauerbehandlung nach einem oder mehreren der vorhergehenden Ansprüche, **gekennzeichnet durch**
einen Vorratsbehälter (24) für einen Dauerbehandlungswirkstoff, eine Dosierpumpe (23), die den Dauerbehandlungswirkstoff aus dem Vorratsbehälter (24) saugt und über eine Impfleitung (26) einer Verwirbelungseinrichtung (27) zuführt, welcher Wasser aus einer Frischwasserleitung (3) zugeführt wird, wobei eine Wasservolumenmeßeinheit (21) die Menge des zugeführten Frischwassers mißt und mengenproportionale Impulse an eine Steuereinheit (22) abgibt, die die Dosierpumpe steuert.
